(19) **Europäisches Patentamt**
**European Patent Office**
**Office européen des brevets**

(11) **EP 2 637 037 A1**

(12) **EUROPEAN PATENT APPLICATION**

(43) Date of publication:
**11.09.2013 Bulletin 2013/37**

(51) Int Cl.:
***G01S 15/89*** *(2006.01)*

(21) Application number: **12172759.8**

(22) Date of filing: **20.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.03.2012 KR 20120024112**

(71) Applicant: **Samsung Medison Co., Ltd.**
**Kangwon-do 250-875 (KR)**

(72) Inventors:
• **Lee, Jun-kum**
**250-875 Gangwon-do (KR)**
• **Hyoun, Dong-gyu**
**250-875 Gangwon-do (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(54) **Method and apparatus for obtaining movement velocity and direction of tissue**

(57)     A method of obtaining a movement velocity and direction of a tissue, the method including: obtaining vector Doppler data from a target by transmitting and receiving an ultrasound signal; and detecting the movement velocity and direction of the tissue included in the target based on the obtained vector Doppler data.

FIG. 1

EP 2 637 037 A1

Printed by Jouve, 75001 PARIS (FR)

**Description**

CROSS-REFERENCE TO RELATED PATENT APPLICATION

This application claims the benefit of Korean Patent Application No. 10-2012-0024112, filed on March 8, 2012, in the Korean Intellectual Property Office, the disclosure of which is incorporated herein in its entirety by reference.

BACKGROUND OF THE INVENTION

1. Field of the Invention

The present invention relates to method and apparatus for obtaining a movement direction and velocity of a tissue included in a target by using the vector Doppler effect.

2. Description of the Related Art

Ultrasound diagnosis apparatuses transmit ultrasound signals from a surface of a human body to be diagnosed toward a predetermined part in the inside of the human body and obtain images related to a cross-section of soft tissue or blood flow by using information regarding ultrasound signals reflected from the predetermined portion.

Ultrasound diagnosis apparatuses have various advantages, including a compact size, low cost, and real-time display. Also, ultrasound diagnosis apparatuses have excellent stability because there is no fear of X-ray exposure, and thus, the ultrasound diagnosis apparatuses are commonly used together with other diagnosis apparatuses, such as computerized tomography (CT) scanners, magnetic resonance imaging (MRI) apparatuses, nuclear medicine diagnosis apparatuses, or the like.

General ultrasound diagnosis apparatuses may detect a velocity of blood flow by using the Doppler effect. However, the velocity of blood flow cannot be precisely measured due to the limitation of angle dependency.

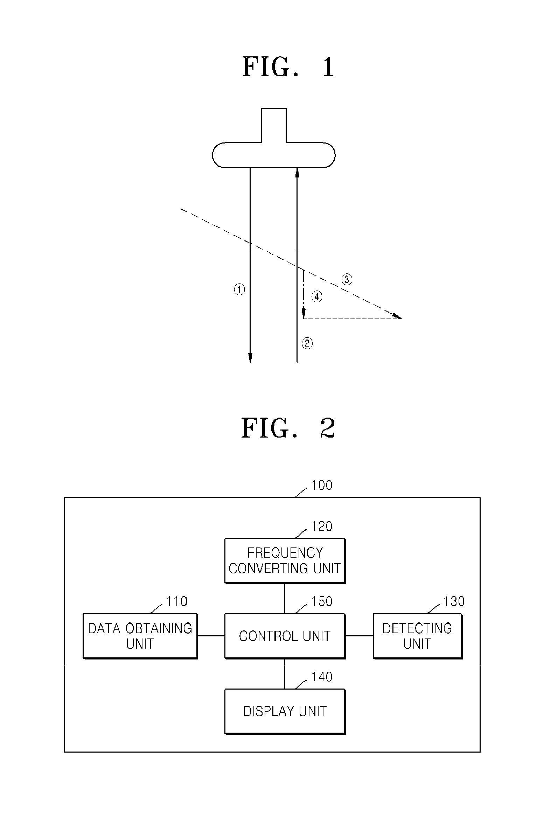

FIG. 1 is a diagram for describing angle dependency of a general ultrasound diagnosis apparatus.

Referring to FIG. 1, a transducer may transmit (a direction ①) an ultrasound signal, and receive (a direction ②) an ultrasound response signal reflected from a target. In this regard, in a case where a movement direction of blood flow is a direction ③, the general ultrasound diagnosis apparatus may not detect that the blood flow precisely moves in the direction ③ due to the limitation of angle dependency. However, the general ultrasound diagnosis apparatus may merely detect that the blood flow moves far away or closer from the transducer. That is, the transducer may recognize only a direction ④ that is an orthogonal component from among movement directions of the blood flow to detect only that the blood flow moves far away from the transducer.

Meanwhile, the general ultrasound diagnosis apparatus may not detect a movement of a cardiac muscle that moves left and right with respect to the transducer due to the angle dependency, and thus the general ultrasound diagnosis apparatus may detect instead a movement of a cardiac valve that moves up and down.

Therefore, an ultrasound diagnosis apparatus for intuitively obtaining a movement direction and velocity of a cardiac muscle without the limitation of angle dependency is needed.

SUMMARY OF THE INVENTION

The present invention provides a method and apparatus for obtaining a movement direction and velocity of a tissue included in a target by using the vector Doppler effect.

According to an aspect of the present invention, there is provided a method of obtaining a movement velocity and direction of a tissue, the method including: obtaining vector Doppler data from a target by transmitting and receiving an ultrasound signal; and detecting the movement velocity and direction of the tissue included in the target based on the obtained vector Doppler data.

The detecting of the movement velocity and direction of the tissue may include: generating frequency spectrum information regarding the obtained vector Doppler data; extracting a clutter signal generated by the tissue based on the generated frequency spectrum information; and detecting the movement velocity and direction of the tissue by using the extracted clutter signal.

The detecting of the movement velocity and direction of the tissue may include: displaying the detected movement velocity and direction of the tissue.

The detecting of the movement velocity and direction of the tissue may further include: calculating the movement velocity and direction of the tissue by using frequencies of at least two clutter signals included in the vector Doppler data.

The extracting of the clutter signal may include: setting a cutoff frequency used to separate a frequency of a blood signal and a frequency of the clutter signal based on the generated frequency spectrum information; and extracting the clutter signal from the vector Doppler data by using a low band filter having the set cutoff frequency.

The extracting of the clutter signal may further include: calculating a gain value based on the frequency spectrum information in such a way that the maximum power of the blood signal has a predetermined value; and extracting the clutter signal by applying the calculated gain value to the vector Doppler data.

The calculated gain value may be greater than 0 and smaller than 1.

The method may further include: compensating for the extracted clutter signal by using a reciprocal of the calculated gain value.

The displaying may include: displaying the movement velocity and direction of the tissue as a two-dimensional (2D) image or a tree-dimensional (3D) image.

The displaying may include: displaying the movement velocity and direction of the tissue in at least one form of colors, particles, and arrows.

The transmission and reception of the ultrasound signal may include at least one of plane wave transmission and reception, focused transmission and reception, scan line transmission and reception, broad beam transmission and reception, and zone based transmission and reception.

According to another aspect of the present invention, there is provided an apparatus for obtaining a movement velocity and direction of a tissue, the apparatus including: a data obtaining unit for obtaining vector Doppler data from a target by transmitting and receiving an ultrasound signal; a detecting unit for detecting the movement velocity and direction of the tissue included in the target based on the obtained vector Doppler data; and a control unit for controlling the data obtaining unit and the detecting unit.

BRIEF DESCRIPTION OF THE DRAWINGS

The above and other features and advantages of the present invention will become more apparent by describing in detail exemplary embodiments thereof with reference to the attached drawings in which:

FIG. 1 is a diagram for describing angle dependency of a general ultrasound diagnosis apparatus;

FIG. 2 is a block diagram of an apparatus for obtaining a movement velocity and direction of a tissue according to an embodiment of the present invention;

FIG. 3 is a flowchart of a method of detecting a movement velocity and direction of a tissue, according to an embodiment of the present invention;

FIG. 4 is a flowchart of a method of detecting a movement velocity and direction of a tissue, according to another embodiment of the present invention;

FIG. 5 is a graph for describing a method of extracting a clutter signal generated by a tissue by using a low band filter, according to an embodiment of the present invention;

FIG. 6 is a graph for describing a method of extracting a clutter signal generated by a tissue by using a gain value, according to an embodiment of the present invention;

FIG. 7 is a diagram for describing a method of obtaining a vector component of a tissue, according to an embodiment of the present invention;

FIG. 8 is a diagram for describing a method of obtaining a vector component of a tissue, according to an embodiment of the present invention; and

FIG. 9 is a diagram for describing a method of a movement velocity and direction of a cardiac muscle, according to an embodiment of the present invention.

DETAILED DESCRIPTION OF THE INVENTION

Most of the terms used herein are general terms that have been widely used in the technical art to which the present invention pertains. However, some of the terms used herein may be created reflecting intentions of technicians in this art, precedents, or new technologies. Also, some of the terms used herein may be arbitrarily chosen by the present applicant. In this case, these terms are defined in detail below. Accordingly, the specific terms used herein should be understood based on the unique meanings thereof and the whole context of the present invention.

In the present specification, it should be understood that the terms, such as 'including' or 'having,' etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added. Also, the terms, such as 'unit' or 'module', etc., should be understood as a unit that processes at least one function or operation and that may be embodied in a hardware manner, a software manner, or a combination of the hardware manner and the software manner.

Throughout the specification, the 'target' may be understood to be part of a human body. For example, the target may be the liver, the heart, the womb, a brain, the breast, or the abdomen, or a fetus.

Throughout the specification, the term 'user' may be a medical professional, e.g., a doctor, a nurse, a clinical pathologist, or a medical imaging professional, but is not limited thereto.

Throughout the specification, a difference between a frequency of an ultrasound signal transmitted to the target and a frequency of an echo signal is referred to as a "Doppler frequency". Further, a signal having the Doppler frequency is referred to as a "Doppler signal".

In the following detailed description, only certain exemplary embodiments of the present invention have been shown and described, simply by way of illustration. As those skilled in the art would realize, the described embodiments may be modified in various different ways, all without departing from the spirit or scope of the present invention. Accordingly, the drawings and description are to be regarded as illustrative in nature and not restrictive. Like reference numerals designate like elements throughout the specification. Expressions such as "at least one of," when preceding a list of elements, modify the entire list of elements and do not modify the individual elements of the list.

FIG. 2 is a block diagram of an apparatus 100 for obtaining a movement velocity and direction of a tissue according to an embodiment of the present invention.

The apparatus 100 for obtaining the movement velocity and direction of the tissue (hereinafter referred to as the "obtaining apparatus 100") according to an embodiment of the present invention may mean a device capable of obtaining an ultrasound image or a vector Doppler image from a target by using ultrasound waves, and displaying the obtained ultrasound image or vector Doppler image to a user.

The obtaining apparatus 100 according to an embodiment of the present invention may be implemented in various ways. For example, the obtaining apparatus 100 described in the present specification may be implemented in a mobile terminal as well as a fixable terminal. Examples of the mobile terminal may include a laptop computer, a PDA, a table PC, and the like.

The obtaining apparatus 100 according to an embodiment of the present invention may include a data obtaining unit 110, a frequency converting unit 120, a detecting unit 130, a display unit 140, and a control unit 150. However, the elements of FIG. 2 are not indispensable elements. The obtaining apparatus 100 may include a greater number of elements or a lesser number of elements.

The data obtaining unit 110 may transmit an ultrasound signal to the target. Also, the data obtaining unit 110 may receive an ultrasound response signal from the target. That is, the data obtaining unit 110 according to an embodiment of the present invention may include a transmitting and receiving unit that transmits and receives the ultrasound signal. An example of the data obtaining unit 110 according to an embodiment of the present invention may include a transducer.

Transmission and reception of the ultrasound signal according to an embodiment of the present invention may include plane wave transmission and reception, focused transmission and reception, scan line transmission and reception, broad beam transmission and reception, and zone based transmission and reception. However, the transmission and reception of the ultrasound signal according to an embodiment of the present invention is not limited thereto.

The data obtaining unit 110 may transmit the ultrasound signal and vector Doppler data from the target. The target according to an embodiment of the present invention may include blood flow and a tissue.

The vector Doppler data according to an embodiment of the present invention may mean Doppler data including a movement direction component of the target. That is, vector Doppler data according to an embodiment of the present invention may include data regarding Doppler signals received from at least two Doppler angles.

The vector Doppler data according to an embodiment of the present invention may include a blood signal generated by the blood flow and a clutter signal generated by the tissue. In this case, according to an embodiment of the present invention, the vector Doppler data may include a plurality of blood signals and a plurality of clutter signals.

The frequency converting unit 120 may generate frequency spectrum information regarding the received vector Doppler data. According to an embodiment of the present invention, the frequency converting unit 120 may generate the frequency spectrum information regarding the received vector Doppler data by using the Fourier transformation.

The frequency spectrum information according to an embodiment of the present invention may include power values (intensity of signals) with respect to frequencies of the blood signal and the clutter signal.

A velocity of the blood flow is relatively faster than that of the tissue, and thus the blood signal generated by the blood flow is located in a high frequency band, and the clutter signal generated by the tissue is located in a low frequency band.

Also, reflectivity of the blood flow is generally quite smaller than that of tissues around the blood flow, and thus the intensity of the clutter signal generated by the tissue is higher than that of the blood signal generated by the blood flow.

The detecting unit 130 may detect a movement velocity and direction of the tissue based on the vector Doppler data. The movement velocity of the tissue according to an embodiment of the present invention may mean the size of a velocity generated by the movement of the tissue. The movement direction of the tissue according to an embodiment of the present invention may mean the direction of the velocity generated by the movement of the tissue.

The detecting unit 130 may extract the clutter signal generated by the tissue based on the generated frequency spectrum information, and detect the movement velocity and direction of the tissue by using the extracted clutter signal. For example, the detecting unit 130 may classify the blood signal and the clutter signal by using the frequency spectrum information of the vector Doppler frequency.

According to an embodiment of the present invention, the detecting unit 130 may classify the blood signal and the clutter signal by using a low band filter. The low band filter may have a cutoff frequency set based on the frequency spectrum information.

The blood signal generated by the blood flow is located in the high frequency band, and the clutter signal generated by the tissue is located in the low frequency band, and thus the detecting unit 130 may extract the clutter signal generated by the tissue by using the low band filter.

According to another embodiment of the present invention, the detecting unit 130 may classify the blood signal and the clutter signal by using a gain value of the vector Doppler data. For example, the detecting unit 130 may calculate the gain value in such a way that the maximum power of the blood signal generated by the blood flow has a predetermined value. According to an embodiment of the present invention, the predetermined value may be "0".

In general, power of the clutter signal generated by the tissue is greater than that of the blood signal generated by the blood flow, and thus the detecting unit 130 may extract the clutter signal generated by the tissue by using the calculated gain value after the power of the blood signal generated by the blood flow is closer to "0".

The detecting unit 130 may obtain the movement velocity and direction of the tissue by using the detected clutter signal generated by the tissue. As described above, the vector Doppler data may include a plurality of clutter signals received from different directions.

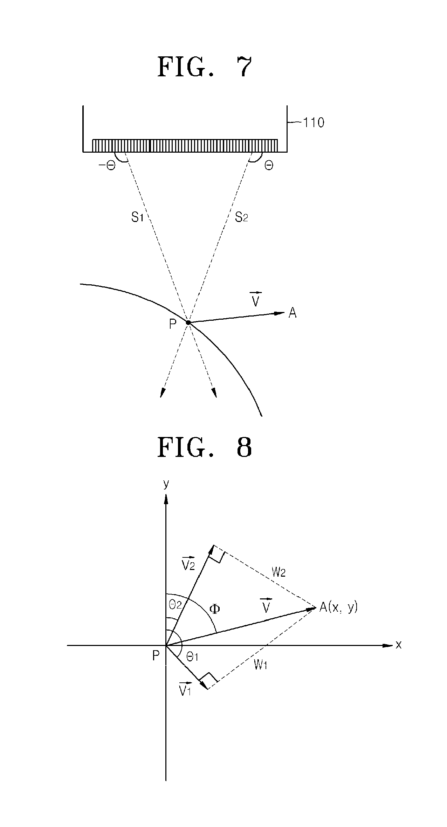

Therefore, the detecting unit 130 may calculate the movement velocity and direction of the tissue based on frequencies of at least two clutter signals. A method of calculating the movement velocity and direction of the tissue will be described in detail with reference to FIG. 7.

The display unit 140 may display and output information processed by the obtaining apparatus 100. For example, the display unit 140 may display the movement velocity and direction of the tissue. In this case, the display unit 140 may overlay and display the movement velocity and direction of the tissue on an ultrasound image of the target.

The ultrasound image according to an embodiment of the present invention may be an image generated through at least one of a brightness (B) mode, a color (C) mode, a motion (M) mode, a pulse-wave (PW) mode, a continuous wave (CW) mode, a two-dimensional (2D) mode, and a three-dimensional (3D) mode.

Meanwhile, the display unit 140 may display the movement velocity and direction of the tissue as a 2D image or a 3D image. The display unit 140 may display the movement velocity and direction of the tissue in at least one form of colors, particles, and arrows.

In a case where the display unit 140 and a touch pad form a mutual layer structure and thus are configured as a touch screen, the display unit 140 may be used as an input device other than an output device. The display unit 140 may include at least one of a liquid crystal display (LCD), a thin film transistor-liquid crystal display (TFT-LCD), an organic light-emitting diode (OLED), a flexible display, and a 3D display.

The obtaining apparatus 100 may also include two or more display units 140. The touch screen may be configured to detect a touch input pressure as well as a touch input location and a touched area. The touch screen may also be configured to detect a proximity touch as well as a real touch.

The control unit 150 may generally control the data obtaining unit 110, the frequency converting unit 120, the detecting unit 130, and the display unit 140.

According to an embodiment of the present invention, the control unit 150 may set the cutoff frequency used to separate the frequency of the blood signal and the frequency of the clutter signal based on the frequency spectrum information generated by the frequency converting unit 120.

The control unit 150 may calculate the gain value in such a way that the maximum power of the blood signal generated by the blood flow has the predetermined value based on the frequency spectrum information generated by the frequency converting unit 120.

Meanwhile, the control unit 150 may compensate for the clutter signal extracted by applying the calculated gain value. In this regard, the control unit 150 compensates for the extracted clutter signal by using a reciprocal number of the calculated gain value.

The obtaining apparatus 100 according to an embodiment of the present invention may transmit and receive unfocused beam. In a case where focused beam is generally used, the obtaining apparatus 100 needs to transmit the focused beam for each scan line, which increases an amount of calculation. However, the obtaining apparatus 100 according to an embodiment of the present invention uses the unfocused beam, which reduces the amount of calculation, thereby detecting the movement velocity and direction of the tissue in real time.

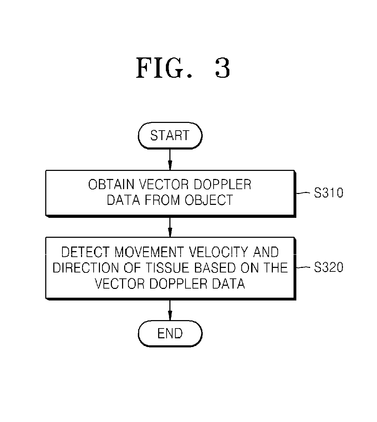

A method of detecting the movement velocity and direction of the tissue by using each element of the obtaining apparatus 100 will be described in detail with reference to FIG. 3.

FIG. 3 is a flowchart of a method of detecting a movement velocity and direction of a tissue, according to an embodiment of the present invention.

Referring to FIG. 3, the method of detecting the movement velocity and direction of the tissue according to an embodiment of the present invention includes operations that are time-serially processed by the obtaining apparatus 100 shown in FIG. 2. Therefore, although omitted, the above description of the obtaining apparatus 100 shown in FIG. 2 also applies to the method shown in FIG. 3.

The obtaining apparatus 100 may transmit and receive an ultrasound signal and obtain vector Doppler data from a target (S310). The target according to an embodiment of the present invention may include blood flow and a tissue. Thus, the vector Doppler data according to an embodiment of the present invention may include a blood signal generated by the blood flow and a clutter signal generated by the tissue.

The obtaining apparatus 100 may detect a movement velocity and direction of the tissue based on the obtained vector Doppler data (S320). The movement velocity and direction of the tissue according to an embodiment of the present invention is information regarding a movement of the target. For example, if the target is a heart, the movement velocity and direction of the tissue may be the size of a velocity of the heart that contracts and relaxes and a direction thereof.

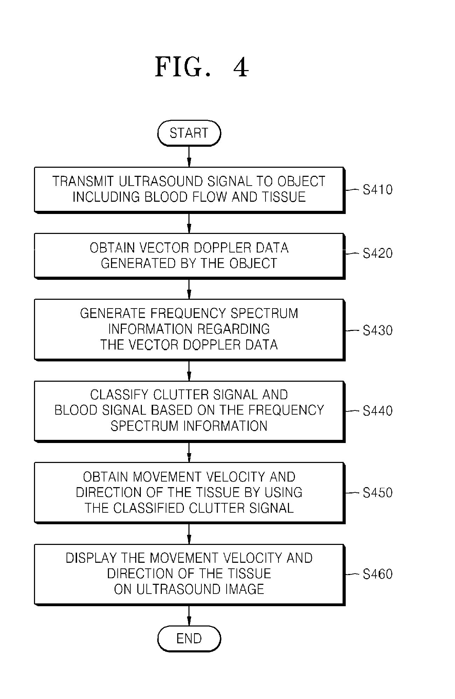

This will be described in more detail with reference to FIG. 4.

FIG. 4 is a flowchart of a method of detecting a movement velocity and direction of a tissue, according to another embodiment of the present invention.

Referring to FIG. 4, the method of detecting the movement velocity and direction of the tissue according to an embodiment of the present invention includes operations that are time-serially processed by the obtaining apparatus 100 shown in FIG. 2. Therefore, although omitted, the above description of the obtaining apparatus 100 shown in FIG. 2 also applies to the method shown in FIG. 4.

The obtaining apparatus 100 may transmit an ultrasound signal to a target including blood flow and a tissue (S41 0). In this case, the obtaining apparatus 100 obtains vector Doppler data generated by the target (S420). The vector Doppler data according to an embodiment of the present invention may include a blood signal generated by the blood flow and a clutter signal generated by the tissue.

According to an embodiment of the present invention, the obtaining apparatus 100 may transmit a plurality of ultrasound signals having different transmission angles to the target and receive a plurality of echo signals having different receiving angles from the target. According to another embodiment of the present invention, the obtaining apparatus 100 may transmit one ultrasound signal to the target and receive a plurality of echo signals having different receiving angles from the target.

The obtaining apparatus 100 may generate frequency spectrum information regarding the vector Doppler data (S430). The frequency spectrum information according to an embodiment of the present invention may indicate correlations between frequencies of the "blood signal and the clutter signal" and intensities thereof.

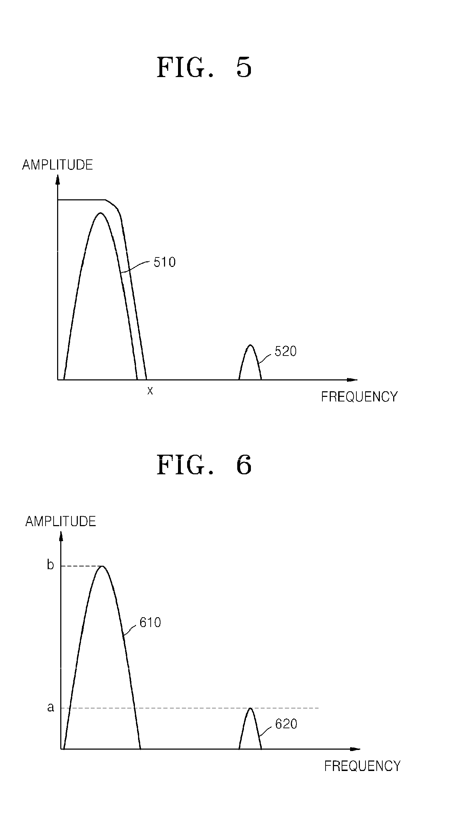

The frequency spectrum information according to an embodiment of the present invention may be expressed as shown in FIG. 5. Referring to FIG. 5, an X axial direction may indicate frequency components of a clutter signal 510 and a blood signal 520, and a Y axial direction may indicate power value (intensities of signals) component of the clutter signal 510 and the blood signal 520.

The obtaining apparatus 100 may extract the clutter signal generated by the tissue by classifying the clutter signal and the blood signal based on the frequency spectrum information (S440).

According to an embodiment of the present invention, the obtaining apparatus 100 may extract the clutter signal by using a low band filter. This will be described with reference to FIG. 5.

FIG. 5 is a graph for describing a method of extracting a clutter signal generated by a tissue by using a low band filter, according to an embodiment of the present invention.

As shown in FIG. 5, a velocity of blood flow is relatively quite faster than that of the tissue, and thus the blood signal 520 generated by the blood flow is located in a high frequency band, and the clutter signal 510 generated by the tissue is located in a low frequency band. In this case, the obtaining apparatus 100 may set a cutoff frequency used to detect the clutter signal based on the frequency spectrum information. That is, the obtaining apparatus 100 may set the cutoff frequency suitable for removing the blood signal 520 generated by the blood flow that is present in the high frequency band. For example, the cutoff frequency may be x in FIG. 5.

In this case, the obtaining apparatus 100 may detect the clutter signal by applying the low band filter having the set cutoff frequency to the vector Doppler data.

According to another embodiment of the present invention, the obtaining apparatus 100 may extract the clutter signal by adjusting a gain value of the vector Doppler data. This will be described with reference to FIG. 6.

FIG. 6 is a graph for describing a method of extracting a clutter signal generated by a tissue by using a gain value, according to an embodiment of the present invention.

In general, reflectivity of blood flow is quite smaller than that of tissues (vessel walls, muscle, etc.) around the blood flow, and thus the intensity of the clutter signal generated by the tissue is mostly higher than that of a blood signal generated by the blood flow.

That is, referring to FIG. 6, according to frequency spectrum information, power of a clutter signal 610 generated by the tissue is much greater than that of a blood signal 620 generated by the blood flow.

Thus, the obtaining apparatus 100 may calculate the gain value in such a way that the maximum power of the blood signal 620 has a predetermined value. According to an embodiment of the present invention, the predetermined value may be closer to "0". Also, the gain value may be greater than 0 and smaller than 1.

That is, the obtaining apparatus 100 may extract the clutter signal 610 by applying the gain value that makes the maximum power of the blood signal 620 closer to "0" to vector Doppler data.

For example, in a case where the maximum power of the blood signal 620 has a value "a", and the maximum power of the clutter signal 610 has a value "b", the obtaining apparatus 100 may calculate the gain value "0.0001" that makes the value "a" of the maximum power of the blood signal 620 closer to "0". If the gain value "0.0001" is applied to the vector Doppler data, the maximum power of the blood signal 620 is a×0.0001 ( 0) closer to "0", and the maximum power of the clutter signal 610 is b×0.0001 ( 0) much greater than "0". Thus, the obtaining apparatus 100 may extract the clutter signal 610 generated by the blood flow.

Meanwhile, the obtaining apparatus 100 may compensate for a gain with respect to the clutter signal detected by adjusting the gain value. That is, the obtaining apparatus 100 may compensate for a power value of the clutter signal by using a reciprocal of the calculated gain value.

For example, in a case where the calculated gain value is "0.0001 ", since the maximum power value of the detected clutter signal is bX0.0001, if the reciprocal "10000(1/0.0001)" of the calculated gain value is applied to the detected clutter signal, the maximum power value of the detected clutter signal is "b".

Referring back to FIG. 3, the obtaining apparatus 100 may obtain the movement velocity and direction of the tissue by using the detected clutter signal (S450). The vector Doppler data according to an embodiment of the present invention may include a plurality of clutter signals having different receiving angles.

Therefore, the obtaining apparatus 100 may calculate the movement velocity and direction of the tissue based on frequencies of the plurality of clutter signals. This will be described with reference to FIGS. 7 and 8.

FIG. 7 is a diagram for describing a method of obtaining a vector component of a tissue, according to an embodiment of the present invention.

The data obtaining unit 110 transmits a first ultrasound signal S1 and a second ultrasound signal S2 to a tissue P of a target. The tissue P moves in a direction A as a velocity vector V. Frequencies of the first ultrasound signal S1 and the second ultrasound signal S2 vary according to the tissue P moving in the direction A. Thus, the data obtaining unit 110 receives an echo signal having a changed frequency.

FIG. 8 is a diagram for describing a method of obtaining a vector component of the tissue P, according to an embodiment of the present invention.

If the velocity vector V of the tissue P is projected in a vertical direction, velocity vectors V1 and V2 may be obtained. The first ultrasound signal S1 is reflected by the tissue P and is transmitted to the data obtaining unit 110. A clutter signal generated at this time corresponds to the velocity vector V1. Also, the second ultrasound signal S2 is reflected by the tissue P and is transmitted to the data obtaining unit 110. A clutter signal generated at this time corresponds to the velocity vector V2.

In a case where an angle difference between a y axis and the velocity vector V1 is θ1, and an angle difference between the y axis and the velocity vector V2 is θ2, equations regarding the velocity vectors V1 and V2 are as follows.

$$\text{Regarding } \vec{V}_1, \quad y = \tan\theta_1 \cdot x \qquad [\text{Equation 1}]$$

$$\text{Regarding } \vec{V}_2, \quad y = \tan\theta_2 \cdot x \qquad [\text{Equation 2}]$$

In a case where velocities of the velocity vectors V1 and V2, are v1 and v2, respectively, first dimensional vertical lines w1 and w2 in perpendicular to the velocity vectors V1 and V2, respectively, are as follows:

$$\text{Regarding } w1, \quad y = -(1/\tan\theta_1)x + v1/\cos\theta_1 \qquad [\text{Equation 3}]$$

$$\text{Regarding } w2, \quad y = -(1/\tan\theta_2)x + v2/\cos\theta_2 \qquad [\text{Equation 4}]$$

The following equation between the velocities v1 and v2 and Doppler frequencies regarding the first ultrasound signal S1 and the second ultrasound signal S2 transmitted from the data obtaining unit 110 is obtained.

$$vn = (\quad fn/f) \cdot c \qquad \text{[Equation 5]}$$

In Equation 5 above, fn denotes a Doppler frequency, f denotes frequencies of the first ultrasound signal S1 and the second ultrasound signal S2, c denotes a sound velocity of the first ultrasound signal S1 and the second ultrasound signal S2, and n denotes an integer.

Although the frequencies of the first ultrasound signal S1 and the second ultrasound signal S2 transmitted to the tissue P has opposite directions, since the frequencies are transmitted in different directions having the same size angle, the following equations between $\theta 1$ and $\theta 2$ are as follows.

$$\theta 1 = \pi - \theta 2 \qquad \text{[Equation 6]}$$

$$\cos\theta 1 = -\cos\theta 2 \qquad \text{[Equation 7]}$$

$$\tan\theta 1 = -\tan\theta 2 \qquad \text{[Equation 8]}$$

If an intersection point of Equations 3 and 4 by using Equations 6, 7, and 8 is calculated, the following equations are obtained.

$$x = \frac{v_1 + v_2}{2} \frac{\sin\theta_2}{\cos^2\theta_2} \qquad \text{[Equation 9]}$$

$$y = \frac{v_2 - v_1}{2\cos\theta_2} \qquad \text{[Equation 10]}$$

In equations 9 and 10 above, x and y denote coordinates of A.

If a velocity v of the velocity vector *V* and an angle $\phi$ of the y axis is calculated by using Equations 9 and 10, the following equations are obtained.

$$v = |\vec{v}| = \sqrt{x^2 + y^2}$$

$$\phi = \tan^{-1}\frac{y}{x}$$

The obtaining apparatus 100 may obtain a movement velocity and direction of the tissue P by using the calculated velocity v of the velocity vector *V* and the angle Φ.

Referring back to FIG. 3, the obtaining apparatus 100 may display the movement velocity and direction of the tissue P on an ultrasound image of the target (S460). According to an embodiment of the present invention, the obtaining apparatus 100 may display the movement velocity and direction of the tissue P as a two-dimensional (2D) image or a three-dimensional (3D) image. The obtaining apparatus 100 may display the movement velocity and direction of the tissue P in at least one form of colors, particles, and arrows.

Therefore, according to an embodiment of the present invention, the obtaining apparatus 100 may precisely detect sizes of the movement direction of velocity of the tissue P without being influenced by angle dependency, and provide a user with the movement velocity and direction of the tissue P.

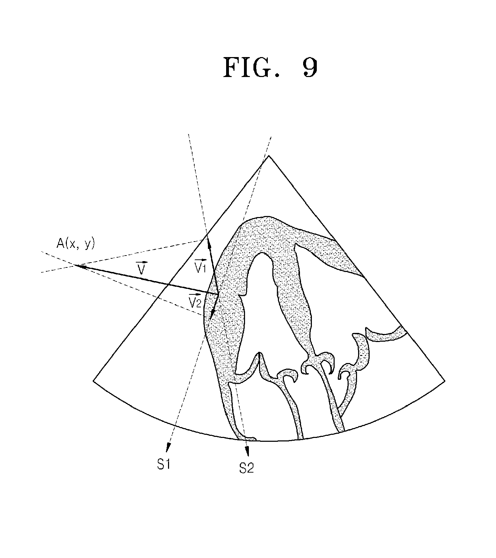

FIG. 9 is a diagram for describing a method of a movement velocity and direction of a cardiac muscle, according to an embodiment of the present invention.

Referring to FIG. 9, even if a user sets sample volume including a tissue (for example, a cardiac muscle) and blood flow, the obtaining apparatus 100 may identify only a clutter signal generated by the tissue (for example, the cardiac muscle) by adjusting a low band filter or a gain value.

The obtaining apparatus 100 also may calculate a movement velocity and direction of the tissue by using frequencies of a plurality of clutter signals generated by the tissue.

Therefore, the obtaining apparatus 100 may display the movement velocity and direction of the tissue on an ultrasound image to the user. That is, according to an embodiment of the present invention, the obtaining apparatus 100 may intuitively present sizes of the movement direction of velocity of the tissue without the limitation of angle dependency.

Embodiments of the present invention include a computer-readable recording medium including program commands for executing operations implemented through various computers. The computer-readable recording medium can store program commands, data files, data structures, or combinations thereof. The program commands recorded in the computer-readable recording medium may be specially designed and configured for the present invention or be known to those of ordinary skill in the field of computer software. Examples of a computer-readable recording medium include magnetic media such as hard disks, floppy disks, and magnetic tapes, optical media such as CD-ROMs and DVDs, magneto-optical media such as floptical disks, or hardware devices such as ROMs, RAMs, and flash memories, which are specially configured to store and execute program commands. Examples of the program commands include a machine language code created by a compiler and a high-level language code executable by a computer using an interpreter and the like.

While the present invention has been particularly shown and described with reference to exemplary embodiments thereof, it will be understood by those of ordinary skill in the art that various changes in form and details may be made therein without departing from the spirit and scope of the present invention as defined by the following claims.

**Claims**

1. A method of obtaining a movement velocity and direction of a tissue, the method comprising:

   obtaining vector Doppler data from a target by transmitting and receiving an ultrasound signal; and
   detecting the movement velocity and direction of the tissue included in the target based on the obtained vector Doppler data.

2. The method of claim 1, wherein the detecting of the movement velocity and direction of the tissue comprises:

   generating frequency spectrum information regarding the obtained vector Doppler data;
   extracting a clutter signal generated by the tissue based on the generated frequency spectrum information; and
   detecting the movement velocity and direction of the tissue by using the extracted clutter signal.

3. The method of claim 2, wherein the detecting of the movement velocity and direction of the tissue comprises:
   displaying the detected movement velocity and direction of the tissue.

4. The method of claim 2, wherein the detecting of the movement velocity and direction of the tissue further comprises:
   calculating the movement velocity and direction of the tissue by using frequencies of at least two clutter signals included in the vector Doppler data.

5. The method of claim 2, wherein the extracting of the clutter signal comprises:

setting a cutoff frequency used to separate a frequency of a blood signal and a frequency of the clutter signal based on the generated frequency spectrum information; and
extracting the clutter signal from the vector Doppler data by using a low band filter having the set cutoff frequency.

6. The method of claim 2, wherein the extracting of the clutter signal further comprises:

calculating a gain value based on the frequency spectrum information in such a way that the maximum power of the blood signal has a predetermined value; and
extracting the clutter signal by applying the calculated gain value to the vector Doppler data.

7. The method of claim 6, further comprising: compensating for the extracted clutter signal by using a reciprocal of the calculated gain value.

8. The method of claim 3, wherein the displaying comprises: displaying the movement velocity and direction of the tissue as a two-dimensional (2D) image or a tree-dimensional (3D) image.

9. The method of claim 3, wherein the displaying comprises: displaying the movement velocity and direction of the tissue in at least one form of colors, particles, and arrows.

10. The method of claim 1, wherein the transmission and reception of the ultrasound signal comprises at least one of plane wave transmission and reception, focused transmission and reception, scan line transmission and reception, broad beam transmission and reception, and zone based transmission and reception.

11. An apparatus for obtaining a movement velocity and direction of a tissue, the apparatus comprising:

a data obtaining unit for obtaining vector Doppler data from a target by transmitting and receiving an ultrasound signal;
a detecting unit for detecting the movement velocity and direction of the tissue included in the target based on the obtained vector Doppler data; and
a control unit for controlling the data obtaining unit and the detecting unit.

12. The apparatus of claim 11, further comprising: a frequency converting unit for generating frequency spectrum information regarding the obtained vector Doppler data,
wherein the detecting unit extracts a clutter signal generated by the tissue based on the generated frequency spectrum information, and detects the movement velocity and direction of the tissue by using the extracted clutter signal.

13. The apparatus of claim 11, further comprising: a display unit for displaying the detected movement velocity and direction of the tissue.

14. The apparatus of claim 12, wherein the detecting unit calculates the movement velocity and direction of the tissue by using frequencies of at least two clutter signals included in the vector Doppler data.

15. The apparatus of claim 12, wherein the control unit sets a cutoff frequency used to separate a frequency of a blood signal and a frequency of the clutter signal based on the generated frequency spectrum information,
wherein the detecting unit extracts the clutter signal from the vector Doppler data by using a low band filter having the set cutoff frequency.

16. The apparatus of claim 12, wherein the control unit calculates a gain value based on the frequency spectrum information in such a way that the maximum power of the blood signal has a predetermined value,
wherein the detecting unit extracts the clutter signal by applying the calculated gain value to the vector Doppler data.

17. The apparatus of claim 13, wherein the display unit displays the movement velocity and direction of the tissue as a two-dimensional (2D) image or a tree-dimensional (3D) image.

18. The apparatus of claim 13, wherein the display unit displays the movement velocity and direction of the tissue in at least one form of colors, particles, and arrows.

**19.** A computer readable recording medium having recorded thereon a program for executing the method of obtaining a movement velocity and direction of a tissue of claim 1.

# FIG. 1

①

②

③

④

# FIG. 2

100

120

FREQUENCY
CONVERTING UNIT

110

DATA OBTAINING
UNIT

150

CONTROL UNIT

130

DETECTING
UNIT

140

DISPLAY UNIT

# FIG. 3

START

OBTAIN VECTOR DOPPLER
DATA FROM OBJECT — S310

DETECT MOVEMENT VELOCITY AND
DIRECTION OF TISSUE BASED ON THE — S320
VECTOR DOPPLER DATA

END

# FIG. 4

```
                    ( START )
                        │
                        ▼
┌─────────────────────────────────────┐
│   TRANSMIT ULTRASOUND SIGNAL TO      │──── S410
│   OBJECT INCLUDING BLOOD FLOW        │
│   AND TISSUE                         │
└─────────────────────────────────────┘
                        │
                        ▼
┌─────────────────────────────────────┐
│   OBTAIN VECTOR DOPPLER DATA         │──── S420
│   GENERATED BY THE OBJECT            │
└─────────────────────────────────────┘
                        │
                        ▼
┌─────────────────────────────────────┐
│   GENERATE FREQUENCY SPECTRUM        │──── S430
│   INFORMATION REGARDING              │
│   THE VECTOR DOPPLER DATA            │
└─────────────────────────────────────┘
                        │
                        ▼
┌─────────────────────────────────────┐
│   CLASSIFY CLUTTER SIGNAL AND        │──── S440
│   BLOOD SIGNAL BASED ON THE          │
│   FREQUENCY SPECTRUM INFORMATION     │
└─────────────────────────────────────┘
                        │
                        ▼
┌─────────────────────────────────────┐
│   OBTAIN MOVEMENT VELOCITY AND       │──── S450
│   DIRECTION OF THE TISSUE BY USING   │
│   THE CLASSIFIED CLUTTER SIGNAL      │
└─────────────────────────────────────┘
                        │
                        ▼
┌─────────────────────────────────────┐
│   DISPLAY THE MOVEMENT VELOCITY AND  │──── S460
│   DIRECTION OF THE TISSUE            │
│   ON ULTRASOUND IMAGE                │
└─────────────────────────────────────┘
                        │
                        ▼
                    ( END )
```

FIG. 5

AMPLITUDE

510

520

x

FREQUENCY

FIG. 6

AMPLITUDE

b

610

a

620

FREQUENCY

# FIG. 7

# FIG. 8

FIG. 9

Europäisches
Patentamt

European
Patent Office

Office européen
des brevets

## EUROPEAN SEARCH REPORT

Application Number

EP 12 17 2759

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 354 556 A1 (MEDISON CO LTD [KR]) 22 October 2003 (2003-10-22) | 1-3, 5-13, 15-19 | INV. G01S15/89 |
| Y | * page 1, line 6 - line 8 * <br> * page 2, line 17 - line 18 * <br> * page 3, line 2 - line 3 * <br> * page 3, line 19 - line 23 * <br> * page 4, line 16 - line 26 * <br> * page 5, line 1 - line 4 * <br> * page 12, line 11 - line 17 * <br> * page 12, line 21 - line 26 * <br> * page 13, line 1 - line 7 * <br> * page 13, line 9 - line 12 * <br> * page 13, line 16 - line 20 * <br> * page 13, line 25 - line 26 * <br> * page 14, line 1 - line 3 * <br> * claims 2-4, 6 * <br> * figures 5, 6 * | 4,14 | |
| Y | WO 98/00719 A2 (B K MEDICAL A S [DK]; JENSEN JOERGEN ARENDT [DK] B K MEDICAL AS [DK];) 8 January 1998 (1998-01-08) <br> * page 3, line 1 - line 7 * <br> * page 3, line 23 - line 27 * | 4,14 | TECHNICAL FIELDS SEARCHED (IPC) <br><br> G01S |
| Y | EP 0 177 942 A2 (FUJITSU LTD [JP]) 16 April 1986 (1986-04-16) <br> * page 1, line 3 - line 12 * <br> * page 1, line 19 - line 33 * <br> * claim 1 * <br> * figure 1 * | 4,14 | |
| Y | US 4 265 126 A (PAPADOFRANGAKIS EMMANUEL ET AL) 5 May 1981 (1981-05-05) <br> * column 1, line 12 - line 21 * | 4,14 | |

-/--

The present search report has been drawn up for all claims

| Place of search | Date of completion of the search | Examiner |
|---|---|---|
| The Hague | 20 November 2012 | Renaudie, Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches
Patentamt**

**European
Patent Office**

**Office européen
des brevets**

## EUROPEAN SEARCH REPORT

Application Number

EP 12 17 2759

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FOX M D: "Multiple crossed-beam ultrasound Doppler velocimetry", IEEE TRANSACTIONS ON SONICS AND ULTRASONICS, IEEE, US, vol. 25, no. 5, 1 September 1978 (1978-09-01), pages 281-286, XP011358495, ISSN: 0018-9537, DOI: 10.1109/T-SU.1978.31028 * column 1 *<br>----- | 4,14 | |

TECHNICAL FIELDS
SEARCHED      (IPC)

The present search report has been drawn up for all claims

| Place of search | Date of completion of the search | Examiner |
|---|---|---|
| The Hague | 20 November 2012 | Renaudie, Cécile |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 12 17 2759

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2012

| Patent document cited in search report | | Publication date | Patent family member(s) | | Publication date |
|---|---|---|---|---|---|
| EP 1354556 | A1 | 22-10-2003 | DE | 60315092 T2 | 10-04-2008 |
| | | | EP | 1354556 A1 | 22-10-2003 |
| | | | JP | 3769550 B2 | 26-04-2006 |
| | | | JP | 2003310615 A | 05-11-2003 |
| | | | KR | 20030082213 A | 22-10-2003 |
| | | | US | 2003199764 A1 | 23-10-2003 |
| WO 9800719 | A2 | 08-01-1998 | AU | 3254897 A | 21-01-1998 |
| | | | DE | 69710725 D1 | 04-04-2002 |
| | | | DE | 69710725 T2 | 21-11-2002 |
| | | | EP | 0909395 A2 | 21-04-1999 |
| | | | JP | 4100709 B2 | 11-06-2008 |
| | | | JP | 2001503853 A | 21-03-2001 |
| | | | US | 6148224 A | 14-11-2000 |
| | | | WO | 9800719 A2 | 08-01-1998 |
| EP 0177942 | A2 | 16-04-1986 | DE | 3585766 D1 | 07-05-1992 |
| | | | EP | 0177942 A2 | 16-04-1986 |
| | | | JP | 1653665 C | 30-03-1992 |
| | | | JP | 3018458 B | 12-03-1991 |
| | | | JP | 61100236 A | 19-05-1986 |
| | | | US | 4693319 A | 15-09-1987 |
| US 4265126 | A | 05-05-1981 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120024112